# EUROPEAN PATENT SPECIFICATION

(11) **EP 4 382 139 B1**
(45) Date of publication and mention of the grant of the patent: **12.11.2025**
(21) Application number: 23175605.7
(22) Date of filing: 26.05.2023
(51) Int. Cl.: G02B 3/00, G02B 5/00, G02B 27/00, F21V 5/00, F21V 23/00, B60R 13/02, B60Q 3/68, A61L 2/10

(54) **SELF-STERILIZING GARNISH STRUCTURE AND SELF-STERILIZING VEHICLE**
SELBSTSTERILISIERENDE GARNIERUNGSSTRUKTUR UND SELBSTSTERILISIERENDES FAHRZEUG
STRUCTURE DE GARNITURE AUTO-STÉRILISANTE ET VÉHICULE AUTO-STÉRILISANT

(30) Priority: 07.12.2022 TW 111147066
(43) Date of publication of application: 12.06.2024
(73) Proprietor: Wistron Corporation, New Taipei City 22181 (TW)
(72) Inventor: SHIAU, Yi-Hau, 22181 New Taipei City (TW); CHIOU, Hsien-Jung, 22181 New Taipei City (TW); CHO, Chu-Shun, 22181 New Taipei City (TW)
(74) Representative: Viering, Jentschura & Partner mbB Patent- und Rechtsanwälte

(56) References cited:
- EP-A1- 3 967 333
- WO-A1-2021/080638
- CN-U- 216 703 003
- KR-A- 20130 044 718
- KR-A- 20220 018 103
- US-A1- 2021 052 757

## Description

### BACKGROUND

### Technical Field

The present invention relates to ultraviolet (UV) sterilization technologies, and in particular, to a self-sterilizing garnish structure and a self-sterilizing vehicle.

### Related Art

Nowadays, with the development of civilization, people have more frequent social activities, but infectious germs or viruses often spread in the social environment. With the social mobility of people, germs or viruses are more easily contacted and attached to other objects. It is common for people to frequently ride or use vehicles due to long-distance movement, which further causes germs or viruses to easily infect the interior of vehicles, and causes further infection risks.

Conventionally, sterilization is mostly carried out by using UV rays. However, to sterilize the internal space of the vehicle, it is usually inconvenient to mount an additional sterilizing device with UV.

KR 20220018103 A discloses a ceiling-type virus removal device comprising a PCB which generates a control signal for sterilizing an object and emitting light; a case having open front and rear ends and including a plurality of ventilation holes provided in the outer periphery thereof; a bracket configured to seal the rear end of the case and having a rear end detachably attached to the ceiling so that the ceiling-type virus removal device can be installed on the ceiling; a light source module which is disposed in the case and includes a hybrid light emitting diode provided on one side of a substrate, wherein an UV light emitting diode that emits ultraviolet light according to a control signal and an LED that emits light are implemented as the hybrid light emitting diode; a quartz lens disposed inside the case, disposed in front of the light source module, and configured to transmit ultraviolet light or light emitted from the light source module; a cover configured to seal the front end of the case and provided with a plurality of ventilation holes in the form of penetrating the front and rear ends and through-holes for allowing ultraviolet rays or light transmitted from the quartz lens to be emitted toward the object; a human body detection sensor which transmits, when detecting a biometric signal of a person to be measured, the biometric signal of the person to be measured to the PCB; an LED indicator configured to operate according to a control signal and output light to indicate a power supply state of the UV light emitting diode; a battery which supplies power to the light source module and the LED indicator according to the control signal; and a heat sink which dissipates heat generated from the PCB, the light source module, and the battery to cool the PCB, the light module, and the battery.

CN 216703003 U discloses an ultraviolet sterilization device comprising a handheld part and an irradiation part, wherein the irradiation part comprises a first shell, a light source module and a display screen module, the first shell comprises a first upper cover and a first lower cover, a transparent surface cover is arranged on the first upper cover and corresponds to the display screen module, and a light outlet hole is arranged on the first lower cover and corresponds to the light source module. The handheld portion comprises a second shell and a battery pack arranged in the second shell, the second shell comprises a second upper cover and a second lower cover, and the second upper cover is provided with a control button. When the ultraviolet sterilization machine works, the hand-held part is held by hands, and the irradiation part is used for aiming at the target surface to sweep the surface back and forth so as to realize the purpose of ultraviolet sterilization.

US 2021052757 A1 discloses a low dose disinfection and control system that utilizes empirical and theoretical data to compare performance, sensor data, stored patterns, historical usage, use intensity indexes over time and tracking information to provide a sophisticated data collection system for disinfection.

Further prior art can be found in EP 3 967 333 A1, KR 1020130044718 A and WO 2021080638 A1.

### SUMMARY

In addition, at present, a display device is used to continuously emits UV from the inside to the outside to sterilize a display surface that a user often touches. Since the emitted UV is only limited to sterilizing the display device itself and the display surface thereof, even if ultraviolet (UV) is applied to a vehicle display, the UV cannot sterilize an internal space of the vehicle in a wide range, and heat energy generated is difficult to dissipate as a result of the vehicle display embedded on the surface of a vehicle body.

According to the present invention, a self-sterilizing garnish structure according to claim 1 and a self-sterilizing vehicle according to claim 9 are provided. Preferred embodiments are further defined in the dependent claims.

In some embodiments, a self-sterilizing garnish structure has a UV sterilization function. The self-sterilizing garnish structure includes a light-transmitting cover, a circuit board, a light source, and a heat-dissipating layer. The circuit board is located below the light-transmitting cover. The light source includes a plurality of light-emitting modules on the circuit board. The plurality of light-emitting modules is arranged between the circuit board and the light-transmitting cover. The heat-dissipating layer is located below the circuit board. A light-outgoing surface of the light source faces the light-transmitting cover, and is configured to emit UV toward the light-transmitting cover.

The self-sterilizing garnish structure further includes a lens layer, and the lens layer is located between the light-transmitting cover and the light source.

In some embodiments, the self-sterilizing garnish structure may further include a self-sterilizing display and an adhesive layer. The self-sterilizing display is arranged on a periphery of the circuit board and below the light-transmitting cover and configured to emit another UV. The self-sterilizing display is bonded to the light-transmitting cover by the adhesive layer.

In some embodiments, the self-sterilizing garnish structure may further include a self-sterilizing display and an adhesive layer. The self-sterilizing display is arranged on a periphery of the circuit board and below the light-transmitting cover and configured to emit another UV. The lens layer is further arranged between the light-transmitting cover and the self-sterilizing display. The self-sterilizing display is bonded to the lens layer by the adhesive layer.

In some embodiments, the self-sterilizing garnish structure may further include a self-sterilizing display and an adhesive layer. The self-sterilizing display is arranged on a periphery of the circuit board and below the light-transmitting cover and configured to emit another UV. The circuit board is a transparent circuit board, and the lens layer and the transparent circuit board are further arranged above the self-sterilizing display. The self-sterilizing display is bonded to the transparent circuit board by the adhesive layer.

In some embodiments, the self-sterilizing garnish structure may further include a self-sterilizing display and a blackened layer. The self-sterilizing display is arranged on a periphery of the circuit board and below the light-transmitting cover and configured to emit another UV. The blackened layer is located on the light-transmitting cover. In some embodiments, the blackened layer can shield the circuit board. In some other embodiments, the blackened layer can shield the circuit board and the self-sterilizing display.

The lens layer includes a first surface and a second surface. The first surface is attached to the light source, the second surface is attached to the light-transmitting cover, and one of the first surface and the second surface is provided with a plurality of surface microstructures.

In some embodiments, the second surface of the lens layer is provided with the plurality of surface microstructures, and each of the surface microstructures is a raised strip.

The first surface of the lens layer is provided with the plurality of surface microstructures, and each of the surface microstructures is a groove.

The lens layer is composed of a plurality of lens blocks, and the plurality of surface microstructures of any two adjacent lens blocks in the plurality of lens blocks extend in different directions.

-

In some embodiments, the lens layer is composed of a plurality of lens blocks, a part of the plurality of lens blocks is provided with the plurality of surface microstructures, and the other part of the plurality of lens blocks is not provided with the plurality of surface microstructures.

In some other embodiments, the lens layer is composed of a plurality of lens blocks, and each of the lens blocks is provided with the plurality of surface microstructures.

In some embodiments, the light-transmitting cover, the circuit board, the heat-dissipating layer, and the lens layer are flexible.

In some embodiments, the light source includes a plurality of light-emitting modules, and the light-emitting modules are arranged on the circuit board.

In some embodiments, a self-sterilizing garnish structure includes a light-transmitting cover, a blackened layer, a circuit board, a light source, a heat-dissipating layer, a black matrix layer, and a lens layer. The blackened layer, is located on the light-transmitting cover. The circuit board is located below the light-transmitting cover. The light source includes a plurality of light-emitting modules on the circuit board. The plurality of light-emitting modules is arranged between the circuit board and the light-transmitting cover. The heat-dissipating layer is located below the circuit board. A light-outgoing surface of the light source faces the light-transmitting cover, and is configured to emit UV toward the light-transmitting cover. The black matrix layer is located on the circuit board, and the plurality of light-emitting modules are located in a plurality of openings extending through the black matrix layer, wherein at least one of the plurality of light-emitting modules is arranged in each of the openings. The lens layer is located between the light-transmitting cover and the plurality of light-emitting modules.
In some embodiments, the self-sterilizing garnish structure may further include a self-sterilizing display. The self-sterilizing display is arranged on a periphery of the circuit board and below the light-transmitting cover and configured to emit another UV.

In some embodiments, the lens layer is located between the light-transmitting cover and the self-sterilizing display.

In some embodiments, a self-sterilizing vehicle includes a vehicle body and any of the above self-sterilizing garnish structures. The vehicle body is provided with a ride space therein. The self-sterilizing garnish structure is located in the ride space and on an inner wall of the vehicle body. The self-sterilizing garnish structure includes a light-transmitting cover, a circuit board, a light source, and a heat-dissipating layer. The circuit board is located below the light-transmitting cover. The light source includes a plurality of light-emitting modules on the circuit board. The plurality of light-emitting modules is arranged between the circuit board and the light-transmitting cover. The heat-dissipating layer is located below the circuit board. A light-outgoing surface of the light source faces the light-transmitting cover, and is configured to emit UV toward the light-transmitting cover.

### BRIEF DESCRIPTION OF THE DRAWINGS

FIG. 1 is a schematic cross-sectional view of a first implementation of a self-sterilizing garnish structure.
FIG. 2 is a schematic cross-sectional view of a second implementation of a self-sterilizing garnish structure.
FIG. 3 is a schematic cross-sectional view of a third implementation of a self-sterilizing garnish structure.
FIG. 4 is a schematic surface view of an embodiment of a lens layer in FIG. 2 or FIG. 3.
FIG. 5 is a schematic surface view of another embodiment of the lens layer in FIG. 2 or FIG. 3.
FIG. 6 is a schematic diagram of an implementation of a lens layer in FIG. 2 or FIG. 3.
FIG. 7 is a schematic cross-sectional view of a fourth implementation of a self-sterilizing garnish structure.
FIG. 8 is a schematic top view of an implementation of a self-sterilizing garnish structure.
FIG. 9 is a schematic cross-sectional view of a fifth implementation of a self-sterilizing garnish structure.
FIG. 10 is a schematic cross-sectional view of a sixth implementation of a self-sterilizing garnish structure.
FIG. 11 is a schematic cross-sectional view of a seventh implementation of a self-sterilizing garnish structure.
FIG. 12 is a schematic cross-sectional view of an eighth implementation of a self-sterilizing garnish structure.
FIG. 13 is a schematic cross-sectional view of a ninth implementation of a self-sterilizing garnish structure.
FIG. 14 is a schematic cross-sectional view of a tenth implementation of a self-sterilizing garnish structure.
FIG. 15 is a schematic cross-sectional view of an eleventh implementation of a self-sterilizing garnish structure.
FIG. 16 is a schematic cross-sectional view of a twelfth implementation of a self-sterilizing garnish structure.
FIG. 17 is a schematic diagram of an implementation of a self-sterilizing vehicle.
FIG. 18 is a schematic diagram showing UV irradiation of an implementation of a self-sterilizing vehicle.

### DETAILED DESCRIPTION

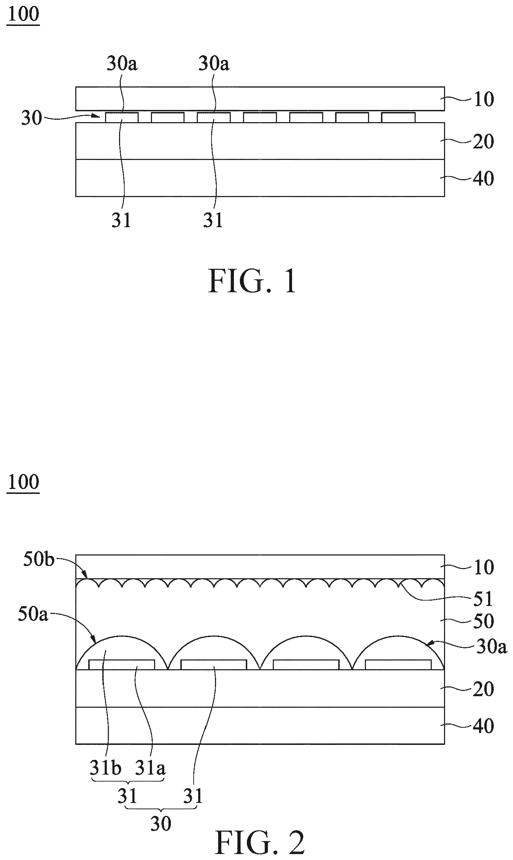

Referring to FIG. 1, a self-sterilizing garnish structure 100 has a UV sterilization function. The self-sterilizing garnish structure 100 includes a light-transmitting cover 10, a circuit board 20, a light source 30, and a heat-dissipating layer 40.

The circuit board 20 is located below the light-transmitting cover 10. In some embodiments, the light-transmitting cover 10 may be made of a material that does not absorb an optical wavelength of UV, or a material that may allow the optical wavelength of UV to penetrate, for example, quartz glass, low boron glass, or plastic. The plastic may be, for example, polypropylene (PP), or the like. Further, the light-transmitting cover 10 is made of a material that does not absorb invisible light (UV) with a wavelength in a range of 100 nm to 400 nm, or that can allow the above wavelength to penetrate.

The light source 30 is arranged between the circuit board 20 and the light-transmitting cover 10 and on the circuit board 20. A light-outgoing surface 30a of the light source 30 faces the light-transmitting cover 10. Therefore, when the light source 30 is driven, the light source 30 emits UV toward the light-transmitting cover 10 for irradiation and sterilization. Further, the light source 30 is arranged on the circuit board 20. In addition, the light source 30 is electrically connected to a circuit wiring on the circuit board 20, and is electrically connected to a driving circuit on the circuit board 20 through the circuit wiring. Therefore, the light source 30 can be driven by the driving circuit to emit UV. In some embodiments, the UV may be UVC. Herein, light with the wavelength range of 100 nm-280 nm and referred to as UVC cannot be irradiated onto the earth, but the light has a plurality of functions such as sterilization, organic decomposition, photopolymerization, sensing, and the like. Therefore, in order to use UVC on the earth, the light source 30 needs to be used to generate artificial UVC. UV irradiation and sterilization have advantages over other sterilization methods. For example, since no chemicals are used, wiping is not required after use of UV. The UV irradiation and sterilization also has the sterilization effect on chlorine-resistant microorganisms that cannot be sterilized by chlorine, and will not produce bacteria resistant to UV sterilization.

The heat-dissipating layer 40 is located below the circuit board 20. Herein, the heat energy generated by the light source 30 is conducted to the heat-dissipating layer 40 through the circuit board 20, and then dissipated by the heat-dissipating layer 40. Therefore, the heat generated by the light source 30 is dissipated by the heat-dissipating layer 40, which can effectively reduce the temperature of the light source 30 and relatively extend the service life of the light source 30. In some embodiments, the heat-dissipating layer 40 may be a copper foil or an artificial graphite plate.

In some embodiments, the light source 30 includes a plurality of light-emitting modules 31, and the light-emitting modules 31 are arranged on the circuit board 20. In some embodiments, the light source 30 may be realized by a UV light-emitting diode (LED) element. For example, each light-emitting module 31 may be a single LED element, or may be composed of a plurality of LED elements, for example, an LED light bar or an LED matrix composed of a plurality of LED elements. A single LED element is used as an example. Each light-emitting module 31 may include an LED chip 31a covered with a condensing lens 31b. Each LED chip 31a emits UV at a light-emitting angle of about 50° to 70°, and the condensing lens 31b converts the UV emitted by the LED chip 31a to UV emitted outward at a light-emitting angle of about 30° or less, so that the UV emitted by the light-emitting module 31 can be irradiated for a long distance. It should be understood that when each light-emitting module 31 includes a plurality of LED elements, the light-emitting module 31 may be composed of an LED light bar or an LED matrix.

In some embodiments, the light-transmitting cover 10, the circuit board 20, and the heat-dissipating layer 40 may be made of flexible materials. Therefore, the whole self-sterilizing garnish structure 100 may be freely arranged on a plane or a non-plane (for example, attached to a curved surface).

Referring to FIG. 2, in some embodiments, the self-sterilizing garnish structure 100 further includes a lens layer 50, and the lens layer 50 is located between the light-transmitting cover 10 and the light source 30. In this embodiment, the light-outgoing surface 30a of the light source 30 faces the lens layer 50, and therefore the UV emitted by the light source 30 enters and passes through the lens layer 50, and then penetrates through the light-transmitting cover 10 to be emitted to the outside of the self-sterilizing garnish structure 100, so that the emitted UV can irradiate and sterilize the outside of the self-sterilizing garnish structure 100. Further, the irradiation angle of the UV emitted by the light source 30 may be changed by the impact of the lens layer 50. Therefore, the UV emitted by the light source 30 is to be emitted from the lens layer 50 at various angles according to the configured lens layer 50, and then penetrates the light-transmitting cover 10 to irradiate and sterilize the outside of the self-sterilizing garnish structure 100. Based on this, the self-sterilizing garnish structure 100 can perform UV irradiation at various angles, to achieve irradiation and sterilization in a wider range. In some embodiments, the lens layer 50 may be made of a material that does not absorb an optical wavelength of UV, or a material that may allow the optical wavelength of UV to penetrate, for example, quartz glass, low boron glass, or plastic. The plastic may be, for example, polypropylene (PP), or the like. Further, the lens layer 50 is made of a material that does not absorb invisible light (UV) with a wavelength in a range of 100 nm to 400 nm, or that can allow the above wavelength to penetrate.

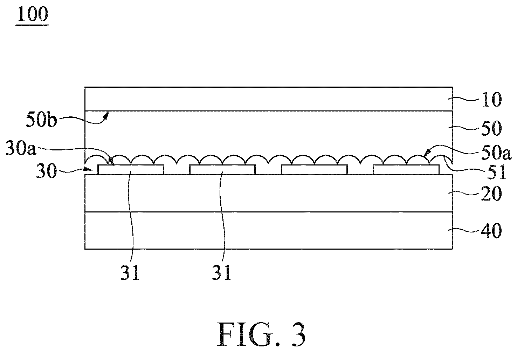

Referring to FIG. 2 or FIG. 3, in some embodiments, the lens layer 50 includes a first surface 50a and a second surface 50b. The first surface 50a is attached to the light source 30, the second surface 50b is attached to the light-transmitting cover 10, and one of the first surface 50a and the second surface 50b is provided with a plurality of surface microstructures 51. In an example, the first surface 50a is provided with the plurality of surface microstructures 51, and each of the surface microstructures 51 is a groove, as shown in FIG. 3. Specifically, the first surface 50a is provided with a plurality of grooves arranged at intervals and extending in the same direction. In this case, the second surface 50b is not provided with the surface microstructure 51. In another example, the second surface 50b is provided with the plurality of surface microstructures 51, and each of the surface microstructures 51 is a raised strip, as shown in FIG. 2. Specifically, the second surface 50b is provided with a plurality of raised strips arranged at intervals and extending in the same direction. In this case, the first surface 50a is not provided with the surface microstructure 51.

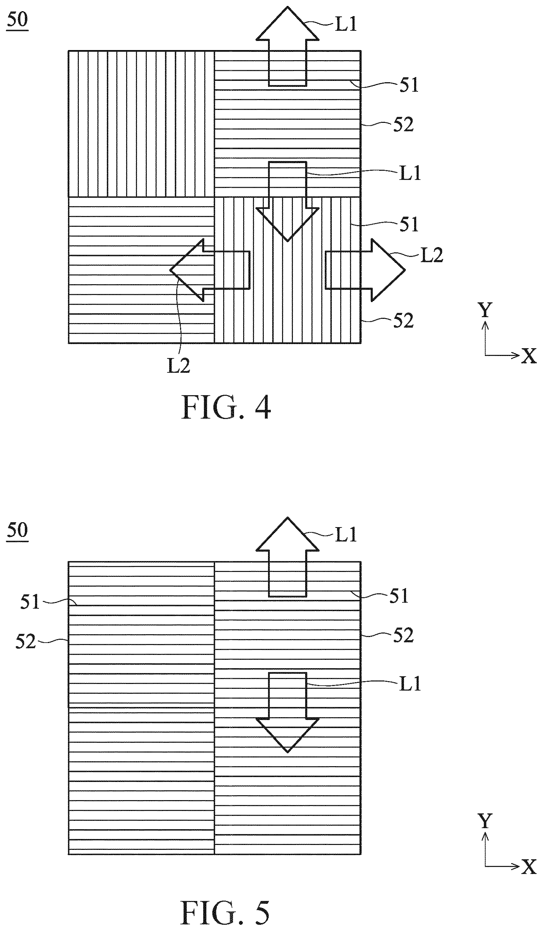

Referring to FIG. 4 to FIG. 6, in some embodiments, the lens layer 50 is composed of a plurality of lens blocks 52.

In some embodiments, the surface microstructures 51 of any two adjacent lens blocks 52 extend in different directions. Herein, with the arrangement of the surface microstructures 51 extending in different directions, the UV emitted by the light source 30 can be simultaneously scattered into UV at various angles in two axial directions. As shown in FIG. 4, the lens block 52 at the upper right is used as an example. UV L1 emitted by the light source 30 may be scattered into the UV L1 at various angles on the Y axis by the impact of the surface microstructure 51. The lens block 52 at the lower right is used as an example. UV L2 emitted by the light source 30 may be scattered into the UV L2 at various angles on the X axis by the impact of the surface microstructure 51. FIG. 4 is used as an example. The surface microstructures 51 are respectively perpendicular or parallel to the horizontal line, but the surface microstructures 51 may also be arranged at different angles. For example, an angle of 45 degrees or other angles are formed between the surface microstructure 51 and the horizontal line (that is, the lens layer 50 in FIG. 4 rotates clockwise or counterclockwise by 45 degrees). Based on this, through the arrangement of different angles formed between the surface microstructure 51 and the horizontal line, the UV emitted by the light source 30 can be scattered into UV at various angles in different axial directions.

Referring to FIG. 5 to FIG. 6, a difference between FIG. 5 and FIG. 4 is that in the embodiment of FIG. 5, the surface microstructures 51 of any two adjacent lens blocks 52 may extend in the same direction. Further, in the embodiment of FIG. 4, the surface microstructures 51 of any two adjacent lens blocks 52 are arranged to extend in different directions, and in the embodiment of FIG. 5, extend in the same direction. In the embodiment of FIG. 5, the surface microstructures 51 of any two adjacent lens blocks 52 may be staggered with respect to each other. Specifically, the surface microstructures 51 of any two adjacent lens blocks 52 do not extend in the same straight line. Based on this, the UV emitted by the light source 30 can be scattered into UV at various angles in an axial direction. As shown in FIG. 5, the lens block 52 at the upper right is used as an example. UV L1 emitted by the light source 30 may be scattered into the UV L1 at various angles on the Y axis by the impact of the surface microstructure 51. FIG. 5 is used as an example. The surface microstructures 51 are parallel to the horizontal line, but the surface microstructures 51 may also be arranged at different angles. For example, an angle of 45 degrees or other angles are formed between the surface microstructure 51 and the horizontal line (that is, the lens layer 50 in FIG. 5 rotates clockwise or counterclockwise by 45 degrees). Based on this, through the arrangement of different angles of the surface microstructure 51, the UV emitted by the light source 30 can be scattered into UV at various angles in different axial directions.

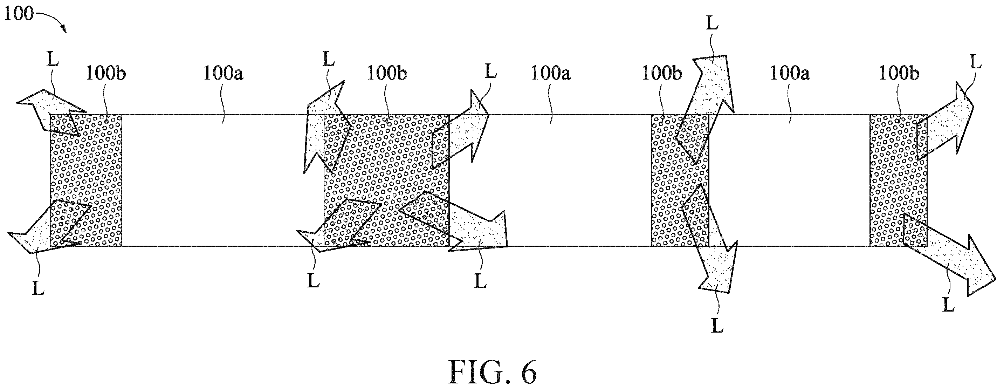

Based on this, the further difference between the embodiment of FIG. 4 and the embodiment of FIG. 5 is that in the embodiment of FIG. 4, the UV emitted by the light source 30 is respectively emitted at various angles on the X axis and the Y axis. In the embodiment of FIG. 5, the UV emitted by the light source 30 is emitted at various angles in the X direction. As shown in the application example of FIG. 6, the self-sterilizing garnish structure 100 can be divided into a display area 100a and a non-display area 100b. When the surface microstructure 51 is applied and arranged according to the embodiment of FIG. 4 or FIG. 5, or various angles are arranged between the surface microstructure 51 and the horizontal line, the UV emitted by the light source 30 can be scattered into UV L at various angles in different axial directions.

In some embodiments, no matter in the embodiment architecture of FIG. 4 or in the embodiment architecture of FIG. 5, only a part of the lens block 52 of the lens layer 50 is provided with a plurality of surface microstructures 51, while the other part of the lens block 52 is not provided with the surface microstructures 51. Further, the lens block 52 with the surface microstructure 51 greatly changes a traveling path of light, while the lens block 52 without the surface microstructure 51 is a pure light-transmitting area, which will not greatly change the traveling path of light (for example, the light is only slightly refracted due to the medium change). In some other embodiments, all of the lens blocks 52 in the lens layer 50 are provided with the surface microstructures 51.

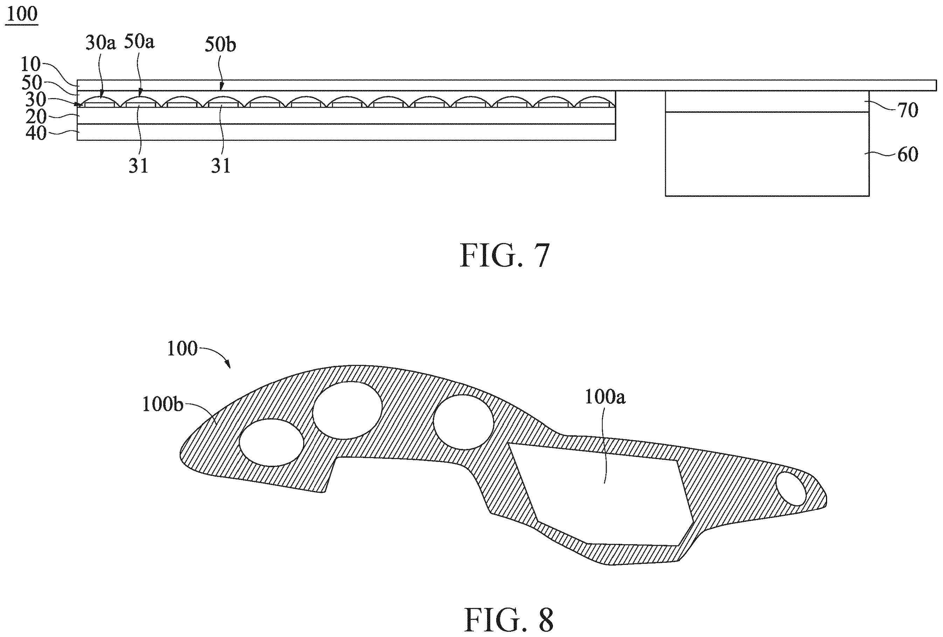

Referring to FIG. 7 and FIG. 8, in some embodiments, the self-sterilizing garnish structure 100 may further include a self-sterilizing display 60. The self-sterilizing display 60 is arranged on a periphery of the circuit board 20 and below the light-transmitting cover 10. The self-sterilizing display 60 can emit another UV for irradiation and sterilization, regardless of whether the self-sterilizing display is performing a display function. Further, the self-sterilizing display 60 may emit another UV from the inside to the outside to irradiate and sterilize the outside of the self-sterilizing display 60 when performing the display function to display an image picture. In another embodiment, the self-sterilizing display 60 can use side-entry light to emit another UV for irradiation and sterilization (that is, an outer surface of the self-sterilizing display 60 is irradiated with the side-entry light). Based on the above, the self-sterilizing garnish structure 100 can be divided into a display area 100a and a non-display area 100b. The display area 100a is an area in which the self-sterilizing display 60 is arranged, and the self-sterilizing display can perform the display function to display the image picture. The non-display area 100b is adjacent to the periphery of the display area 100a (in some embodiments, that is, the area in which the circuit board 20 is arranged).

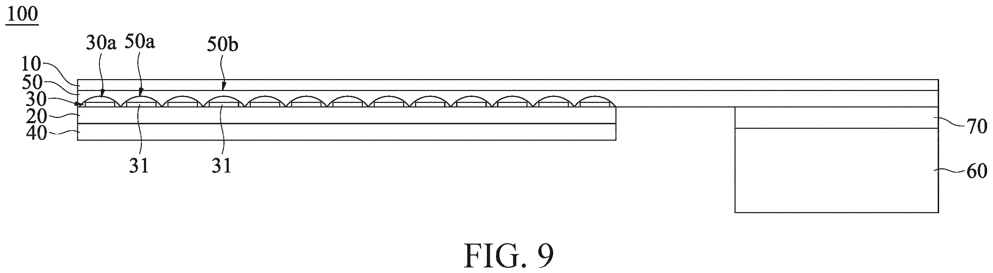

In some embodiments, the self-sterilizing garnish structure 100 further includes an adhesive layer 70. The self-sterilizing display 60 is bonded to the light-transmitting cover 10 by the adhesive layer 70. Specifically, a display surface of the self-sterilizing display 60 is bonded to a lower surface of the light-transmitting cover 10 via the adhesive layer 70. Referring to FIG. 9, in some other embodiments, the lens layer 50 is further arranged between the light-transmitting cover 10 and the self-sterilizing display 60. In other words, part of the lens layer 50 is sandwiched between the light-transmitting cover 10 and the light source 30 (and the circuit board 20), while the other part of the lens layer 50 is sandwiched between the light-transmitting cover 10 and the self-sterilizing display 60. In some embodiments, the self-sterilizing display 60 is bonded to the lens layer 50 by the adhesive layer 70. Specifically, the display surface of the self-sterilizing display 60 is bonded to a lower surface of the lens layer 50 via the adhesive layer 70. An upper surface of the lens layer 50 is attached to the lower surface of the light-transmitting cover 10.

In this embodiment, the lens block 52 sandwiched between the light-transmitting cover 10 and the self-sterilizing display 60 in the lens layer 50 is not provided with the surface microstructure 51 as described above. Therefore, the image displayed by the self-sterilizing display 60 will not be changed by the impact of the lens layer 50.

In some embodiments, the adhesive layer 70 may be an optical adhesive, for example, an optical clear adhesive (OCA, an optical double-sided adhesive tape) or an optical clear resin (OCR, an aqueous adhesive).

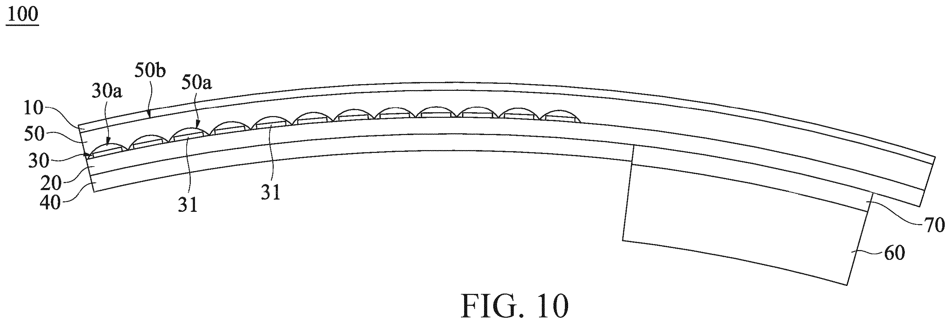

Referring to FIG. 10, in some embodiments, the circuit board 20 is a transparent circuit board, and the lens layer 50 and the transparent circuit board are further arranged above the self-sterilizing display 60. Specifically, the self-sterilizing display 60 is attached to a lower surface of the circuit board 20 together with the heat-dissipating layer 40. Based on this, the image displayed by the self-sterilizing display 60 is to penetrate through the transparent circuit board and is normally displayed. In this embodiment, the self-sterilizing display 60 and the transparent circuit board (the circuit board 20) can be bonded by the adhesive layer 70.

In this embodiment, the light-transmitting cover 10, the circuit board 20, the heat-dissipating layer 40, and the lens layer 50 may be made of flexible materials. Therefore, the self-sterilizing garnish structure 100 may generally present an arc shape.

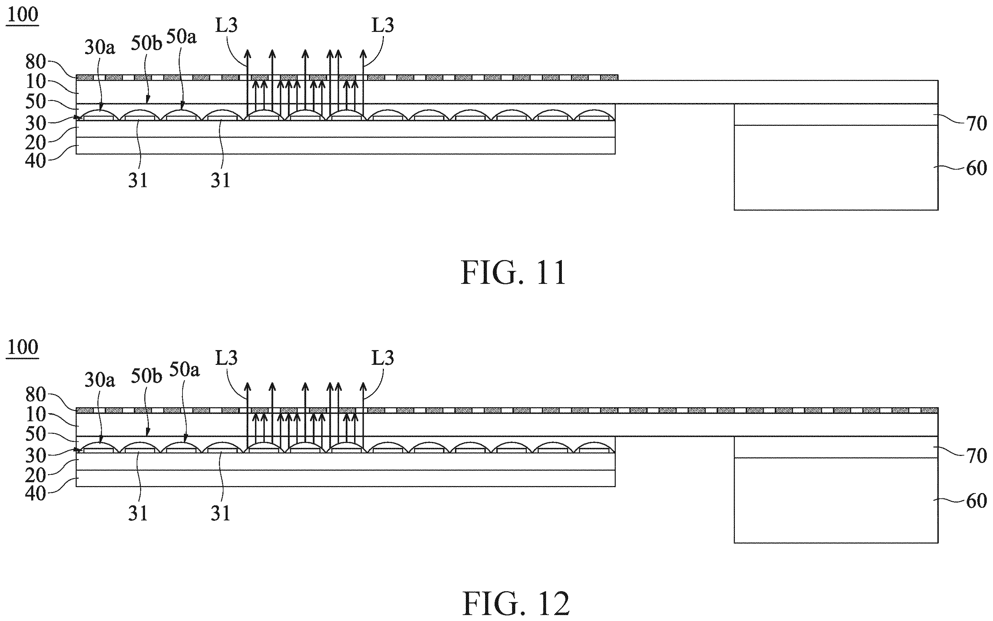

Referring to FIG. 11 and FIG. 12, in an embodiment, the self-sterilizing garnish structure 100 further includes a blackened layer 80. The blackened layer 80 is arranged on the light-transmitting cover 10. In this way, the blackened layer 80 can shield lower components, and the UV can partially penetrate the blackened layer 80 (described in detail later). Based on this, the self-sterilizing garnish structure 100 can reduce the visibility of the lower components (such as the light-emitting module 31) by using the blackened layer 80.

In some embodiments, the blackened layer 80 shields the circuit board 20, as shown in FIG. 11. In some other embodiments, the blackened layer 80 shields the circuit board 20 and the self-sterilizing display 60, as shown in FIG. 12. Further, as shown in FIG. 11, the blackened layer 80 is located on the light-transmitting cover 10 above the circuit board 20, but no blackened layer 80 is arranged on the light-transmitting cover 10 above the self-sterilizing display 60. Based on this, when the light source 30 and the self-sterilizing display 60 do not emit light, when the self-sterilizing garnish structure 100 is viewed from the front of the self-sterilizing garnish structure 100, since no blackened layer 80 is arranged above the self-sterilizing display 60, the appearance of the self-sterilizing display 60 and the appearance above the circuit board 20 will present a visual sense with a slight color difference.

In some embodiments, the blackened layer 80 shields the circuit board 20 and the self-sterilizing display 60, as shown in FIG. 12. Further, the blackened layer 80 is located on the light-transmitting cover 10 above the circuit board 20 and the self-sterilizing display 60. For example, the blackened layer 80 is arranged on the entire upper surface of the light-transmitting cover 10. Based on this, when the light source 30 and the self-sterilizing display 60 do not emit light, the self-sterilizing garnish structure 100 will generally present uniform black when viewed from the front of the self-sterilizing garnish structure 100.

In some embodiments, the blackened layer 80 may be a coating formed by using a light-shielding material formed by carbon black or black pigment mixed with resin. The blackened layer 80 can form a coating with a dense pattern by screen printing or photolithography. In some embodiments, the dense pattern may be a dense pattern with a plurality of dots or squares. In addition, an area with a plurality of dots or squares is an area without the light-shielding material. Based on this, the coating of the dense pattern can shield the components below the dense pattern, and the UV can also partially penetrate the area with the plurality of dots or squares (the area without the light-shielding material). As shown in FIG. 11 or FIG. 12, after the blackened layer 80 forms the coating with the dense pattern by screen printing or photolithography, the dense pattern with the plurality of dots or squares has no light-shielding material. Therefore, after the light source 30 emits UV L3, part of the UV L3 is blocked by the blackened layer 80 (the area with the light-shielding material). The other part of the UV L3 can penetrate the area with the plurality of dots or squares on the blackened layer 80.

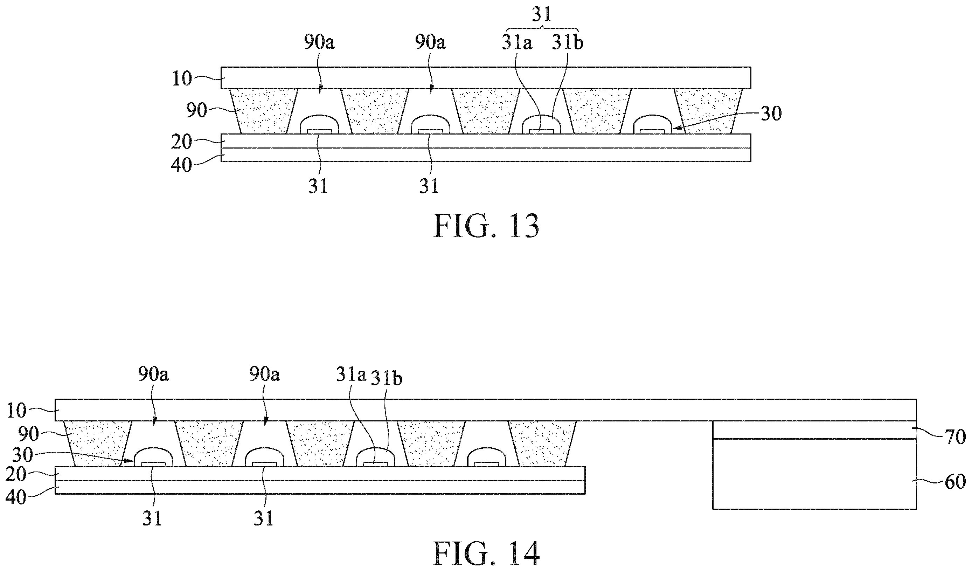

Referring to FIG. 13, the self-sterilizing garnish structure 100 includes a light-transmitting cover 10, a circuit board 20, a light source 30, and a heat-dissipating layer 40. The light source 30 includes a plurality of light-emitting modules 31, and the light-emitting modules 31 are arranged on the circuit board 20. In some embodiments, the self-sterilizing garnish structure 100 further includes a black matrix layer 90. The black matrix layer 90 is located on the circuit board 20, and the light-emitting modules 31 are located in a plurality of openings 90a extending through the black matrix layer 90. In other words, the black matrix layer 90 is formed on the circuit board 20 between the light-emitting modules 31, that is, the black matrix layer 90 and the light-emitting modules 31 are together staggered on the circuit board 20 with respect to each other. In this way, the black matrix layer 90 can reduce the visibility of the light-emitting module 31. In other words, the light-shielding pattern of the black matrix layer 90 can shield the thin and small light-emitting module 31, but the UV emitted by the light-emitting module 31 can pass through the opening 90a. Therefore, if the black matrix layer 90 is made of black light-shielding material, when the light-emitting module 31 does not emit light, the light-transmitting cover 10 can have a garnish effect like black glass when the self-sterilizing garnish structure 100 is viewed from the front of the self-sterilizing garnish structure 100. It should be understood that, in addition to the black light-shielding material, the black matrix layer 90 can actually adopt light-shielding materials of other colors (such as blue, green, gray, and the like) as required. However, the specification adopted by the black matrix layer 90 mentioned above, may be, for example, but not limited to, a thickness between 27 µm and 33 µm (30 µm +/- 3µm ), an optical density of the black matrix layer is less than 1.5, and UVC transmittance is greater than 50%. Furthermore, an outline structure of the black matrix layer 90 mainly adopts a hexagonal structure, and can also adopt a micro-cup structure or a circular structure.

In some embodiments, at least one of the plurality of light-emitting modules 31 is arranged in each of the openings 90a. Further, at least one light-emitting module 31 or the plurality of light-emitting modules 31 are arranged in the each opening 90a.

In some embodiments, viewed from above, a shape of the opening 90a may be cupshaped, circular, or rectangular.

In some embodiments, when the self-sterilizing garnish structure 100 adopts the black matrix layer 90, the lens layer 50 and/or the blackened layer 80 may or may not be selected and used for the self-sterilizing garnish structure 100.

Referring to FIG. 14, in some embodiments, the self-sterilizing garnish structure 100 further includes a self-sterilizing display 60. The self-sterilizing display 60 is arranged on a periphery of the circuit board 20 and below the light-transmitting cover 10. Specifically, the self-sterilizing display 60 is arranged on the lower surface of the light-transmitting cover 10 together with the black matrix layer 90. The self-sterilizing display 60 can emit another UV for irradiation and sterilization when not performing display. In some embodiments, the self-sterilizing garnish structure 100 may be bonded to the light-transmitting cover 10 by the adhesive layer 70.

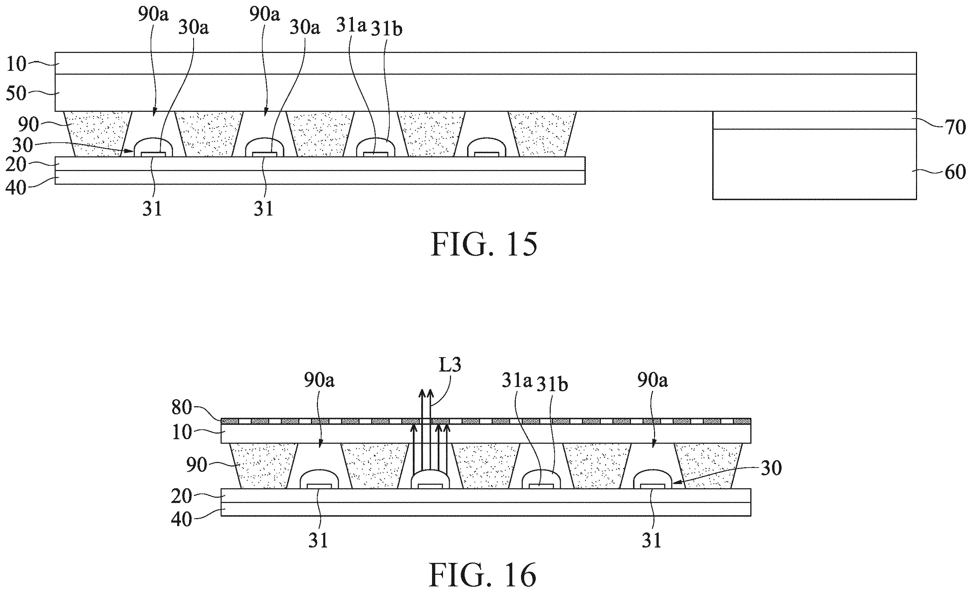

Referring to FIG. 15, in some embodiments, the self-sterilizing garnish structure 100 may further include a lens layer 50, and the lens layer 50 is located between the light-transmitting cover 10 and the plurality of light-emitting modules 31. Specifically, the lens layer 50 is arranged on the lower surface of the light-transmitting cover 10, and the black matrix layer 90 is arranged on the lower surface of the lens layer 50.

In some embodiments, when the self-sterilizing garnish structure 100 is provided with the self-sterilizing display 60, the self-sterilizing display 60 can be directly arranged on the lower surface of the light-transmitting cover 10, that is, the lens layer 50 does not extend above the self-sterilizing display 60.

In some other embodiments, when the self-sterilizing garnish structure 100 is provided with the self-sterilizing display 60, the lens layer 50 may extend above the self-sterilizing display 60, that is, the lens layer 50 is located between the light-transmitting cover 10 and the self-sterilizing display 60, as shown in FIG. 15. Specifically, the self-sterilizing display 60 is arranged on the lower surface of the lens layer 50 together with the black matrix layer 90. The lens layer 50 is located above the black matrix layer 90 and the plurality of light-emitting modules 31. In some embodiments, the self-sterilizing garnish structure 100 may be bonded to the lens layer 50 by the adhesive layer 70.

Referring to FIG. 16, in some embodiments, the self-sterilizing garnish structure 100 may further include a blackened layer 80, and the blackened layer 80 is located on the light-transmitting cover 10. In this way, the blackened layer 80 can shield lower components, but does not block the UV, that is, the UV can penetrate the blackened layer 80. Based on this, the self-sterilizing garnish structure 100 not only can reduce the visibility of internal components (such as the light-emitting module 31) by using the black matrix layer 90, but also can further reduce the visibility of the internal components (such as the light-emitting module 31) by using the blackened layer 80.

In some embodiments, the blackened layer 80 is arranged on the entire upper surface of the light-transmitting cover 10. In some other embodiments, the blackened layer 80 may also be arranged only on a position on the upper surface of the light-transmitting cover 10 corresponding to the circuit board 20. For example, when the self-sterilizing garnish structure 100 is provided with the self-sterilizing display 60, the blackened layer 80 is not arranged above the self-sterilizing display 60, that is, the upper surface of the light-transmitting cover 10 above the self-sterilizing display 60 is not provided with the blackened layer 80.

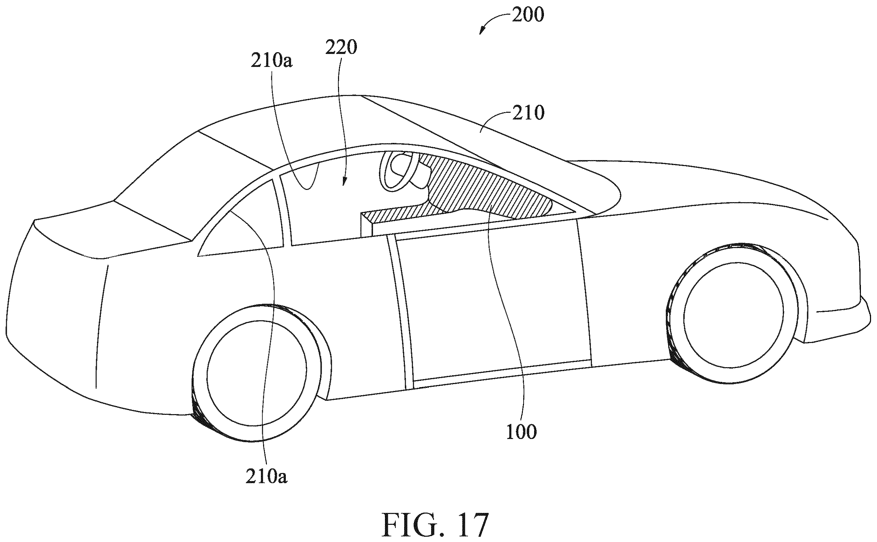

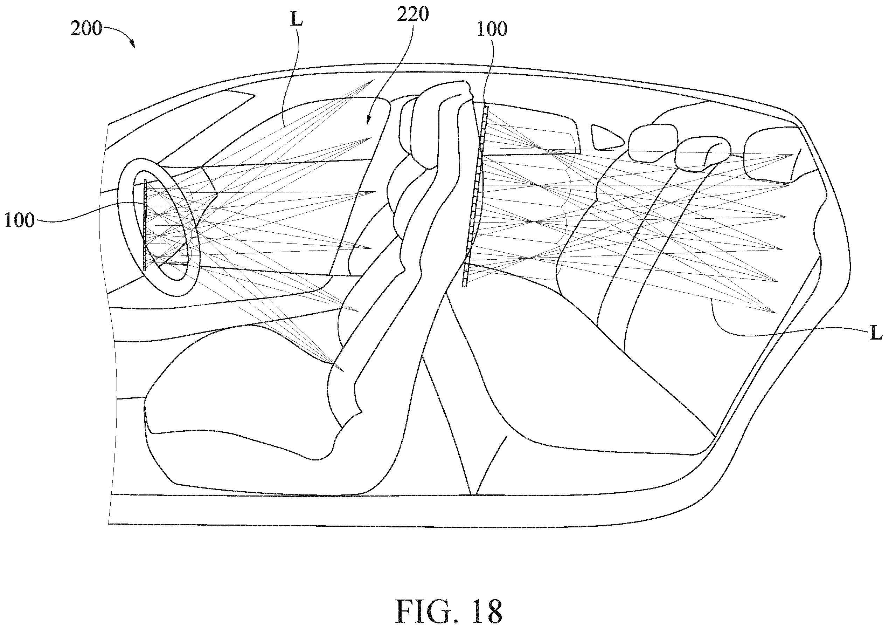

Refer to FIG. 17 and FIG. 18. The self-sterilizing garnish structure 100 of any of the foregoing embodiments can be applied to a vehicle (such as a vehicle, a ship, an airplane, or the like) to irradiate and sterilize the internal space (that is, a ride space 220 and/or the interior) of the vehicle. Specifically, a self-sterilizing vehicle 200 includes a vehicle body 210 and the self-sterilizing garnish structure 100 of any of the foregoing embodiments. The ride space 220 is arranged inside the vehicle body 210. The self-sterilizing garnish structure 100 is located in the ride space 220 and on an inner wall 210a of the vehicle body 210. In other words, the self-sterilizing garnish structure 100 may be embedded in any position on the inner wall 210a of the vehicle body 210. The vehicle body 210 is a domestic or commercial vehicle for riding by way of example. The inner wall 210a of the vehicle body 210 is any surface around the ride space 220 in the vehicle, for example, a roof inside the vehicle, an underbody inside the vehicle, a back rest in the vehicle (as shown in FIG. 18, the self-sterilizing garnish structure 100 is arranged on the back rest of a front seat of the vehicle), an inner side surface of the vehicle door, a dashboard of the vehicle (as shown in FIG. 18, the self-sterilizing garnish structure 100 is arranged on the dashboard of the vehicle), or the like. Therefore, the detailed structure of the self-sterilizing garnish structure 100 is as described above, and the details will not be described again.

For example, the self-sterilizing garnish structure 100 can be applied to a part other than the display screen of the front dashboard of the vehicle, that is, as a panel of the front dashboard of the vehicle. In another example, the self-sterilizing garnish structure 100 can be embedded in a center console on a side of a driver seat of the vehicle. As shown in the schematic diagram of UV irradiation of FIG. 18, UV L emitted from the self-sterilizing garnish structure 100 can be emitted from the vehicle body 210 at various angles. In other words, the UV L emitted from the self-sterilizing garnish structure 100 can be irradiated to different positions in different directions, and can be irradiated to the driver seat or the assistant seat, the steering wheel, and the internal garnish interior of each vehicle for a long distance. When the self-sterilizing garnish structure 100 is arranged on the back of the front seat of a vehicle, the UV can irradiate different positions such as the rear seat of the vehicle in different directions. In other words, the ride space 220 of the vehicle body 210 can be irradiated and sterilized.

In some other application examples, the self-sterilizing garnish structure 100 of any embodiment can be self-sterilized after the car is off or when the ride space in the car is unoccupied. That is, the self-sterilizing function can be enabled in a passive way. That is to say, the self-sterilizing garnish structure 100 turns on the UV to self-sterilize the ride space 220 and/or the interior (such as the front seat and the rear seat in the car) when no one is around.

Based on the above, the self-sterilizing garnish structure 100 or the self-sterilizing vehicle 200 of any embodiment is applicable to a vehicle with the ride space 220 (such as a vehicle, a ship, an airplane, and the like), which continuously emits UV to irradiate and sterilize the ride space 220, and quickly takes away heat energy generated by the UV light source 30 through the heat-dissipating layer 40, so as to avoid greatly reducing the service life of the light source 30. In some embodiments, the self-sterilizing garnish structure 100 or the self-sterilizing vehicle 200 not only can sterilize the surface of the self-sterilizing garnish structure 100, but also can irradiate and sterilize the inner space (that is, the ride space 220 and/or the interior) of the vehicle body 210 in a long distance and/or in a large range.

## Claims

1. A self-sterilizing garnish structure, comprising:
a light-transmitting cover (10);
a circuit board (20), arranged below the light-transmitting cover (10);
a light source (30), comprising a plurality of light-emitting modules (31) being on the circuit board (20) and arranged between the circuit board (20) and the light-transmitting cover (10), wherein a light-outgoing surface (30a) of the light source (30) faces the light-transmitting cover (10), and is configured to emit ultraviolet (UV) toward the light-transmitting cover (10);
a lens layer (50), arranged between the light-transmitting cover (10) and the light source (30); and
a heat-dissipating layer (40), arranged below the circuit board (20),
wherein the lens layer (50) comprises a first surface (50a) and a second surface (50b), the first surface (50a) is attached to the light source (30), the second surface (50b) is attached to the light-transmitting cover (10), and one of the first surface (50a) and the second surface (50b) is provided with a plurality of surface microstructures (51), **characterized in that**
the first surface (50a) is provided with the plurality of surface microstructures (51), and each of the surface microstructures (51) is a groove, the lens layer (50) is composed of a plurality of lens blocks (52), and the plurality of surface microstructures (51) of any two adjacent lens blocks (52) in the plurality of lens blocks (52) extend in different directions, wherein the plurality of surface microstructures (51) are arranged in parallel in the same lens block (52), and in perpendicular configuration in adjacent lens blocks.

2. The self-sterilizing garnish structure according to claim 1, further comprising:
a self-sterilizing display (60), arranged on a periphery of the circuit board (20) and below the light-transmitting cover (10) to emit another UV; and
an adhesive layer (70), wherein the self-sterilizing display (60) is bonded to the light-transmitting cover (10) by the adhesive layer (70).

3. The self-sterilizing garnish structure according to any of claims 1 and 2, further comprising:
a self-sterilizing display (60), arranged on a periphery of the circuit board (20) and below the light-transmitting cover (10) to emit another UV, wherein the lens layer (50) is further arranged between the light-transmitting cover (10) and the self-sterilizing display (60); and
an adhesive layer (70), wherein the self-sterilizing display (60) is bonded to the lens layer (50) by the adhesive layer (70).

4. The self-sterilizing garnish structure according to any of claims 1-3, further comprising:
a self-sterilizing display (60), arranged on a periphery of the circuit board (20) and below the light-transmitting cover (10) to emit another UV, wherein the circuit board (20) is a transparent circuit board, and the lens layer (50) and the transparent circuit board are further arranged above the self-sterilizing display (60); and
an adhesive layer (70), wherein the self-sterilizing display (60) is bonded to the transparent circuit board by the adhesive layer (70).

5. The self-sterilizing garnish structure according to any of the preceding claims, further comprising:
a self-sterilizing display (60), arranged on a periphery of the circuit board (20) and below the light-transmitting cover (10) to emit another UV; and
a blackened layer (80), arranged on the light-transmitting cover (10), wherein the blackened layer (80) shields the circuit board (20) or the circuit board (20) and the self-sterilizing display (60).

6. The self-sterilizing garnish structure according to any of the preceding claims, wherein the second surface (50b) is provided with the plurality of surface microstructures (51), and each of the surface microstructures (51) is a raised strip.

7. The self-sterilizing garnish structure according to any of the preceding claims, wherein the lens layer (50) is composed of a plurality of lens blocks (52), a part of the plurality of lens blocks (52) is provided with the plurality of surface microstructures (51), and the other part of the plurality of lens blocks (52) is not provided with the plurality of surface microstructures (51).

8. The self-sterilizing garnish structure according to claim 1, further comprising:
a blackened layer (80), arranged on the light-transmitting cover (10); and
a black matrix layer (90), arranged on the circuit board (20), wherein the plurality of light-emitting modules (31) are arranged in a plurality of openings (90a) extending through the black matrix layer (90), wherein at least one of the plurality of light-emitting modules (31) is arranged in each of the openings (90a);
wherein the lens layer (50) is arranged between the light-transmitting cover (10) and the plurality of light-emitting modules (31).

9. A self-sterilizing vehicle, comprising:
a vehicle body (210), provided with a ride space (220) therein; and
the self-sterilizing garnish structure (100) according to claim 1, arranged in the ride space (220) and on an inner wall (210a) of the vehicle body (210).

## Patentansprüche

1. Selbststerilisierendes-Zierelement-Aufbau, aufweisend:
eine lichttransmittierende Abdeckung (10);
eine Leiterplatte (20), welche unterhalb von der lichttransmittierenden Abdeckung (10) angeordnet ist;
eine Lichtquelle (30), welche mehrere lichtemittierende Module (31) aufweist, welche sich an der Leiterplatte (20) befinden und zwischen der Leiterplatte (20) und der lichttransmittierenden Abdeckung (10) angeordnet sind, wobei eine Lichtaustrittsfläche (30a) der Lichtquelle (30) der lichttransmittierenden Abdeckung (10) zugewandt ist und konfiguriert ist, um Ultraviolett (UV) in Richtung hin zu der lichttransmittierenden Abdeckung (10) zu emittieren;
eine Linsenschicht (50), welche zwischen der lichttransmittierenden Abdeckung (10) und der Lichtquelle (30) angeordnet ist;
eine Wärmeableitungsschicht (40), welche unterhalb von der Leiterplatte (20) angeordnet ist,
wobei die Linsenschicht (50) eine erste Fläche (50a) und eine zweite Fläche (50b) aufweist, wobei die erste Fläche (50a) an der Lichtquelle (30) angebracht ist, wobei die zweite Fläche (50b) an der lichttransmittierenden Abdeckung (10) angebracht ist, und wobei eine von der ersten Fläche (50a) und der zweiten Fläche (50b) mit mehreren Oberflächenmikrostrukturen (51) versehen ist,
**dadurch gekennzeichnet, dass**:
die erste Fläche (50a) mit den mehreren Oberflächenmikrostrukturen (51) versehen ist, und jede von den Oberflächenmikrostrukturen (51) eine Vertiefung ist, wobei die Linsenschicht (50) aus mehreren Linsenblöcken (52) zusammengesetzt ist, und sich die mehreren Oberflächenmikrostrukturen (51) von zwei beliebigen benachbarten Linsenblöcken (52) in den mehreren Linsenblöcken (52) in unterschiedliche Richtungen erstrecken, wobei die mehreren Oberflächenmikrostrukturen (51) in demselben Linsenblock (52) parallel und in benachbarten Linsenblöcken in senkrechter Konfiguration angeordnet sind.

2. Selbststerilisierendes-Zierelement-Aufbau gemäß Anspruch 1, ferner aufweisend:
einen selbststerilisierenden Bildschirm (60), welcher an einem Randbereich der Leiterplatte (20) und unterhalb von der lichtemittierenden Abdeckung (10) angeordnet ist, um ein anderes UV zu emittieren; und
eine Klebstoffschicht (70), wobei der selbststerilisierende Bildschirm (60) mittels der Klebstoffschicht (70) an die lichttransmittierende Abdeckung (10) gebondet ist.

3. Selbststerilisierendes-Zierelement-Aufbau gemäß einem der Ansprüche 1 und 2, ferner aufweisend:
einen selbststerilisierenden Bildschirm (60), welcher an einem Randbereich der Leiterplatte (20) und unterhalb von der lichttransmittierenden Abdeckung (10) angeordnet ist, um ein anderes UV zu emittieren, wobei die Linsenschicht (50) ferner zwischen der lichttransmittierenden Abdeckung (10) und dem selbststerilisierenden Bildschirm (60) angeordnet ist; und
eine Klebstoffschicht (70), wobei der selbststerilisierende Bildschirm (60) mittels der Klebstoffschicht (70) an die Linsenschicht (50) gebondet ist.

4. Selbststerilisierendes-Zierelement-Aufbau gemäß einem der Ansprüche 1 bis 3, ferner aufweisend:
einen selbststerilisierenden Bildschirm (60), welcher an einem Randbereich der Leiterplatte (20) und unterhalb von der lichttransmittierenden Abdeckung (10) angeordnet ist, um ein anderes UV zu emittieren, wobei die Leiterplatte (20) eine transparente Leiterplatte ist, und wobei die Linsenschicht (50) und die transparente Leiterplatte ferner oberhalb von dem selbststerilisierenden Bildschirm (60) angeordnet sind; und
eine Klebstoffschicht (70), wobei der selbststerilisierende Bildschirm (60) mittels der Klebstoffschicht (70) an die transparente Leiterplatte gebondet ist.

5. Selbststerilisierendes-Zierelement-Aufbau gemäß einem der vorhergehenden Ansprüche, ferner aufweisend:
einen selbststerilisierenden Bildschirm (60), welcher an einem Randbereich der Leiterplatte (20) und unterhalb von der lichttransmittierenden Abdeckung (10) angeordnet ist, um ein anderes UV zu emittieren; und
eine geschwärzte Schicht (80), welche an der lichttransmittierenden Abdeckung (10) angeordnet ist, wobei die geschwärzte Schicht (80) die Leiterplatte (20) oder die Leiterplatte (20) und den selbststerilisierenden Bildschirm (60) abschirmt.

6. Selbststerilisierendes-Zierelement-Aufbau gemäß einem der vorhergehenden Ansprüche, wobei die zweite Fläche (50b) mit den mehreren Oberflächenmikrostrukturen (51) versehen ist, und jede von den Oberflächenmikrostrukturen (51) ein erhöhter Streifen ist.

7. Selbststerilisierendes-Zierelement-Aufbau gemäß einem der vorhergehenden Ansprüche, wobei die Linsenschicht (50) aus mehreren Linsenblöcken (52) zusammengesetzt ist, ein Teil der mehreren Linsenblöcke (52) mit den mehreren Oberflächenmikrostrukturen (51) versehen ist, und der andere Teil der mehreren Linsenblöcke (52) nicht mit den mehreren Oberflächenmikrostrukturen (51) versehen ist.

8. Selbststerilisierendes-Zierelement-Aufbau gemäß Anspruch 1, ferner aufweisend:
eine geschwärzte Schicht (80), welche an der lichttransmittierenden Abdeckung (10) angeordnet ist; und
eine Schwarze-Matrix-Schicht (90), welche an der Leiterplatte (20) angeordnet ist, wobei die mehreren lichtemittierenden Module (31) in mehreren Öffnungen (90a), welche sich durch die Schwarze-Matrix-Schicht (90) hindurch erstrecken, angeordnet sind, wobei mindestens eines von den mehreren lichtemittierenden Modulen (31) in jeder von den Öffnungen (90a) angeordnet ist;
wobei die Linsenschicht (50) zwischen der lichttransmittierenden Abdeckung (10) und den mehreren licht emittierenden Modulen (31) angeordnet ist.

9. Selbststerilisierendes Fahrzeug, aufweisend:
einen Fahrzeugkörper (210), welcher mit einem Fahrgastraum (220) darin versehen ist; und
den Selbststerilisierendes-Zierelement-Aufbau (100) gemäß Anspruch 1, welcher in dem Fahrgastraum (220) und an einer Innenseite (210a) des Fahrzeugkörpers (210) angeordnet ist.

## Revendications

1. Structure de garniture auto-stérilisante, comprenant :
un couvercle transmettant la lumière (10) ;
une carte de circuit (20), disposée sous le couvercle transmettant la lumière (10) ;
une source lumineuse (30), comprenant une pluralité de modules électroluminescents (31) situés sur la carte de circuit (20) et disposés entre la carte de circuit (20) et le couvercle transmettant la lumière (10), dans laquelle une surface de sortie de lumière (30a) de la source lumineuse (30) fait face au couvercle transmettant la lumière (10), et est configurée pour émettre un rayonnement ultraviolet (UV) vers le couvercle transmettant la lumière (10) ;
une couche de lentille (50), disposée entre le couvercle transmettant la lumière (10) et la source lumineuse (30) ; et
une couche de dissipation de chaleur (40), disposée sous la carte de circuit (20),
dans laquelle la couche de lentille (50) comprend une première surface (50a) et une deuxième surface (50b), la première surface (50a) est fixée à la source lumineuse (30), la deuxième surface (50b) est fixée au couvercle transmettant la lumière (10), et l'une parmi la première surface (50a) et la deuxième surface (50b) est pourvue d'une pluralité de microstructures de surface (51), **caractérisée en ce que** la première surface (50a) est pourvue de la pluralité de microstructures de surface (51), et chacune des microstructures de surface (51) est une rainure, la couche de lentille (50) est composée d'une pluralité de blocs de lentilles (52), et la pluralité de microstructures de surface (51) de deux blocs de lentilles adjacents (52) quelconques dans la pluralité de blocs de lentilles (52) s'étendent dans des directions différentes, dans laquelle la pluralité de microstructures de surface (51) sont disposées en parallèle dans le même bloc de lentilles (52), et dans une configuration perpendiculaire dans des blocs de lentilles adjacents.

2. Structure de garniture auto-stérilisante selon la revendication 1, comprenant en outre :
un affichage auto-stérilisant (60), disposé sur une périphérie de la carte de circuit (20) et sous le couvercle transmettant la lumière (10) pour émettre un autre rayonnement UV ; et
une couche adhésive (70), dans laquelle l'affichage auto-stérilisant (60) est lié au couvercle transmettant la lumière (10) par la couche adhésive (70).

3. Structure de garniture auto-stérilisante selon l'une quelconque des revendications 1 et 2, comprenant en outre :
un affichage auto-stérilisant (60), disposé sur une périphérie de la carte de circuit (20) et sous le couvercle transmettant la lumière (10) pour émettre un autre rayonnement UV, dans laquelle la couche de lentille (50) est en outre disposée entre le couvercle transmettant la lumière (10) et l'affichage auto-stérilisant (60) ; et
une couche adhésive (70), dans laquelle l'affichage auto-stérilisant (60) est lié à la couche de lentille (50) par la couche adhésive (70).

4. Structure de garniture auto-stérilisante selon l'une quelconque des revendications 1 à 3, comprenant en outre :
un affichage auto-stérilisant (60), disposé sur une périphérie de la carte de circuit (20) et sous le couvercle transmettant la lumière (10) pour émettre un autre rayonnement UV, dans laquelle la carte de circuit (20) est une carte de circuit transparente, et la couche de lentille (50) et la carte de circuit transparente sont en outre disposées au-dessus de l'affichage auto-stérilisant (60) ; et
une couche adhésive (70), dans laquelle l'affichage auto-stérilisant (60) est lié à la carte de circuit transparente par la couche adhésive (70).

5. Structure de garniture auto-stérilisante selon l'une quelconque des revendications précédentes, comprenant en outre :
un affichage auto-stérilisant (60), disposé sur une périphérie de la carte de circuit (20) et sous le couvercle transmettant la lumière (10) pour émettre un autre rayonnement UV ; et
une couche noircie (80), disposée sur le couvercle transmettant la lumière (10), dans laquelle la couche noircie (80) protège la carte de circuit (20) ou la carte de circuit (20) et l'affichage auto-stérilisant (60).

6. Structure de garniture auto-stérilisante selon l'une quelconque des revendications précédentes, dans laquelle la deuxième surface (50b) est pourvue de la pluralité de microstructures de surface (51), et chacune des microstructures de surface (51) est une bande en relief.

7. Structure de garniture auto-stérilisante selon l'une quelconque des revendications précédentes, dans laquelle la couche de lentille (50) est composée d'une pluralité de blocs de lentilles (52), une partie de la pluralité de blocs de lentilles (52) est pourvue de la pluralité de microstructures de surface (51), et l'autre partie de la pluralité de blocs de lentilles (52) n'est pas pourvue de la pluralité de microstructures de surface (51).

8. Structure de garniture auto-stérilisante selon la revendication 1, comprenant en outre :
une couche noircie (80), disposée sur le couvercle transmettant la lumière (10) ; et
une couche matricielle noire (90), disposée sur la carte de circuit (20), dans laquelle la pluralité de modules électroluminescents (31) sont disposés dans une pluralité d'ouvertures (90a) s'étendant à travers la couche matricielle noire (90), dans laquelle au moins un de la pluralité de modules électroluminescents (31) est disposé dans chacune des ouvertures (90a) ;
dans laquelle la couche de lentille (50) est disposée entre le couvercle transmettant la lumière (10) et la pluralité de modules électroluminescents (31).

9. Véhicule auto-stérilisant, comprenant :
une carrosserie de véhicule (210), pourvue d'un espace de conduite (220) à l'intérieur ; et
la structure de garniture auto-stérilisante (100) selon la revendication 1, disposée dans l'espace de conduite (220) et sur une paroi intérieure (210a) de la carrosserie de véhicule (210).
